Europäisches Patentamt

European Patent Office

Office européen des brevets

(11) Publication number: **0 199 318 B1**

# EUROPEAN PATENT SPECIFICATION

(45) Date of publication of patent specification: **03.03.93**   (51) Int. Cl.⁵: **B29C 41/14**

(21) Application number: **86105456.7**

(22) Date of filing: **02.09.83**

(60) Publication number of the earlier application in accordance with Art.76 EPC: **0 105 613**

(54) **Dipped rubber article.**

(30) Priority: **03.09.82 GB 8225200**
**30.11.82 US 445436**

(43) Date of publication of application:
**29.10.86 Bulletin 86/44**

(45) Publication of the grant of the patent:
**03.03.93 Bulletin 93/09**

(84) Designated Contracting States:
**AT BE CH DE FR GB IT LI LU NL SE**

(56) References cited:
**WO-A-81/00345        FR-A- 1 434 453**
**FR-A- 2 193 710       US-A- 3 813 695**
**US-A- 3 959 554       US-A- 4 143 109**

(73) Proprietor: **LRC PRODUCTS LIMITED**
**North Circular Road**
**London, E4 8OA(GB)**

(72) Inventor: **James, Michael Howard**
**Oakleigh House 12 West Common Way**
**Harpenden Hertfordshire(GB)**
Inventor: **Bratby, David Michael**
**18 Deborah Court Victoria Road**
**South Woodford London E.18(GB)**
Inventor: **Blackley, David Charles**
**100 Berkeley Avenue Chesham**
**Buckinghamshire HP5 2RS(GB)**
Inventor: **Duck, Roger**
**29 Woodstock Road**
**Upper Walthamstow London E17(GB)**
Inventor: **Podell, Howard Irwin**
**28 Beachfront Lane**
**New Rochelle New York 10805(US)**
Inventor: **Goldstein, Albert**
**87 Glenwood Drive**
**Tinton Falls New Jersey 07724(US)**

(74) Representative: **Spencer, Graham Easdale et al**
**A.A. Thornton & CO Northumberland House**
**303-306, High Holborn**
**London WC1V 7LE (GB)**

## Description

The present invention is concerned with flexible rubber articles and, in particular, thin-walled rubber gloves of the kind used by surgeons.

Surgeon's gloves are difficult to don and to facilitate donning a powdered lubricant, such as particulate epichlorhydrin-treated maize starch, is conventionally applied to the inner surface of the gloves. There is a risk of such powered lubricant escaping from the interior of the glove to contaminate the surgical field, the lubricant escaping either during donning or, as sometimes happens, if the glove is punctured during an operation.

Proposals have been made for polymeric lubricant coatings which are bonded to the inner surface of such gloves and which, because they are bonded, cannot escape from the glove.

Examples of such proposals are in U.S. Patents 4070713 and 4143109, which disclose gloves which have an inner layer of elastomeric material with particulate lubricant embedded therein, and U.S. Patents 3813695, 3856561 and 4302852, which disclose surgeon's gloves with various polymeric slip coatings bonded to the inner surface thereof.

U.S. Patent 3813695 ("the Podell patent") describes a surgeon's glove in which the glove material is formed of a laminate consisting of an outer layer of flexible material, for example rubber, and an inner layer of hydrophilic plastic material (such as a hydrogel polymer), the inner and outer layers being bonded together.

There are many known hydrogel polymers, examples of which are polyvinyl pyrrolidone, polyhydroxyethyl acrylate or methacrylate, polyhydroxypropyl acrylate or methacrylate, and copolymers of these with each other or with acrylic or methacrylic acid, acrylic or methacrylic esters or vinyl pyridine.

There are many disclosures of the coating of rubber articles, such as catheters and bathing caps, with such hydrogel polymers by dipping in a solution of a hydrophilic, hydrogel-forming polymer and curing the resulting polymer layer.

Examples of such disclosures include U.S. Patents 3326742, 3585103, 3607473, 3745042, 3901755, 3925138, 3930076, 3940533, 3966530, 4024317, 4110495 and 4125477, and British Patents 1028446 and 859297.

A further similar disclosure is French Patent 1,434,453 which describes a process for making rubber gloves having a slippery surface formed from a mixture of an elastomer latex and a latex of a resin, such as an acrylic resin, for example methyl or ethyl acrylate or methacrylate polymers.

We have evaluated many hydrogel polymers and have surprisingly found that certain 2-hydroxyethyl methacrylate polymers provide superior lubricity with respect to dry skin and can be appropriately treated to provide superior lubricity with respect to damp skin.

According to the present invention, therefore, there is provided a flexible rubber article having a lubricating layer formed from a hydrogel polymer bonded thereto so as to provide a skin-contacting surface of said article (as described, for example, in U.S. Patent 3,813,695 referred to above), which article is characterised in that the hydrogel polymer is a copolymer of 2-hydroxyethyl methacrylate (HEMA) with methacrylic acid (MAA) or with 2-ethylhexyl acrylate (EHA), or a ternary copolymer of HEMA, MAA, and EHA, said copolymer having a composition falling within the area ABCDEF of the attached ternary composition diagram. The area ABCDEF is believed to cover substantially all of the hydrogel-forming area of the composition diagram, apart from HEMA homopolymer and HEMA copolymers with up to 5% EHA and/or MAA.

The composition is preferably within area ABXYZ, more preferably within area PQRI. It is most preferred that the composition is within area IJKL.

In some embodiments, such a copolymer may contain HEMA and MAA in a molar ratio of at least 1:1 (such as 1 to 10:1) or HEMA and EHA in a molar ratio of at least 2.5:1 (such as 2.5 to 10:1).

The copolymer, which is preferably prepared by solution polymerisation (bulk polymerisation is less satisfactory), may be a binary copolymer of HEMA and MAA or EHA, or it may be a ternary copolymer of these monomers with EHA; a preferred such terpolymer has a monomer molar ratio of HEMA to (MAA + EHA) of 67:33 to 90:10 (that is 2 to 9:1) and a molar ratio of EHA to (HEMA + MAA) of 5:97 to 20:80 (that is 1: about 20 to 4).

Minor amounts of further monomers which do not impair the properties of the copolymer may be used in addition. A mixture of such copolymers can be employed, either with other such copolymers or with minor amounts of other polymers which do not impair the properties of the hydrogel-forming polymer.

It is to be noted that the EHA is of a hydrophobic nature and assists in the bonding of the hydrogel polymer to the (hydrophobic) rubber substrate. EHA also serves as a plasticiser and increases the flexibility of the layer formed from the hydrogel polymer (which is bonded to a flexible rubber substrate). EHA may accordingly be replaced by an alkyl acrylate or methacrylate which acts in a corresponding manner.

The MAA serves to furnish cross-linking sites in the copolymer; the methyl group in MAA may influence the copolymer by

(i) modifying the reactivity of the carboxyl groups and thus influencing the rate and degree of cross-linking, and

(ii) by modifying the rate at which MAA is incorporated into the polymer backbone and hence the distribution of cross-linking sites along the polymer chain.

Copolymers as described above provide better lubricity with respect to dry skin than any other type of hydrogel-forming polymer we have evaluated. Surface treatment of such coatings derived from these and other hydrogel-forming polymers to provide lubricity with respect to damp skin is described in our Application 105613 (83305099.0).

The article according to the invention is preferably treated with a silicone liquid so as to reduce the surface tack on any surfaces not coated with a layer formed from the hydrogel polymer. It is preferred that treatment with a silicone (such as medical grade polydimethyl siloxane) is carried out with a bath containing at least 0.05% by weight of silicone (for example, 0.05 to 0.4% by weight).

The rubber used in the article according to the present invention may be a natural or synthetic rubber; natural rubber is preferred. It is also preferred that the article according to the invention should be formed (prior to bonding of the layer formed from hydrogel polymer thereto) by dipping of a rubber latex. The hydrogel layer is preferably applied to the rubber before vulcanisation thereof; this has the suprising result that the skin-contacting surface has a much cooler feel. This may be because there is greater vapour transmission through the final coated article.

An article according to the invention can be produced by a process comprising the steps of:

(a) forming a rubber article by dipping a suitable shaped former in a rubber latex,

(b) leaching the rubber article on the former in hot water,

(c) priming the rubber surface of the article on the former, for example by means of a dilute acid,

(d) rinsing the primed surface in water or aqueous alkali,

(e) dipping said article, while still on the former, in a solution of a hydrophilic, hydrogel-forming polymer and a curing agent therefor,

(f) heating the resulting coating so that the resulting hydrogel polymer is bonded to the rubber and so that the rubber is vulcanised and the polymer is simultaneously cured, and

(g) stripping the resulting article from the former.

This process may optionally comprise the additional steps of:

(h) applying a solution of surfactant material containing silicone to the article (for example, by tumbling in such a solution), optionally after washing, and

(i) heating the resulting coating of surfactant material so as to fix the slip properties of the coating.

Our tests have shown that, following step (i), the damp skin slip properties and the dry skin slip properties of the coating formed from the hydrogel polymer are advantageously not impaired by subsequent washing (that is, surfactant material is not leached out to any substantial extent on washing of the coated article).

The application of the solution of surfactant material provides a substantially tack-free outer surface (that is, the surface not coated withhydrogel polymer), in addition to the inner surface, which is of course, advantageous.

The rubber surface to which the hydrogel polymer is bonded may also be primed by dipping in, for example, a solution of an aluminium salt after priming with dilute acid.

It is a feature of the present invention that the production of the dipped rubber article, leaching, priming, application of hydrogel polymer layer, and vulcanisation of the rubber and curing of the polymer can all be carried out in a continuous operation.

The present invention has been described primarily, with reference to surgeons' gloves; it is, however, applicable to other skin- or tissue-contacting flexible rubber articles, such as condoms, gloves used by doctors and veterinary surgeons for examination purposes (such gloves being often donned with dry hands), catheters, urethers, sheets and sheath-type incontinence devices.

When the present invention is used for articles such as urethers and catheters, the layer formed from hydrogel polymer is provided on the outer surface (this being the skin-contacting surface); for condoms the layer formed from hydrogel polymer may be provided on the inner surface and/or on the outer surface.

In order that the present invention may be more fully understood, the following Examples and Comparative Examples are given by way of illustration only.

3

Example 1

A thin dipped surgeons glove of natural rubber latex was leached with sulphuric acid, rinsed, primed by dipping in aluminium sulphate solution, dried out completely and then dipped into a 4% alcoholic solution of a copolymer of 2-hydroxyethyl methacrylate (HEMA) and methacrylic acid (MAA) in a 1:1 molar ratio, followed by drying. The solution contained, in addition to the copolymer, 5 parts per hundred of partially methylated melamine-formaldehyde resin (as cross-linking agent) and 0.5 parts per hundred of paratoluene sulphonic acid (as catalyst).

The rubber was then vulcanised, after which the lubricity with respect to dry skin was subjectively evaluated on a scale of 1 to 5, in which:

1 means that the film is sticky

2 means that poor slip is obtained

3 means that moderate slip is obtained

4 means that quite good slip is obtained

5 means that excellent slip is obtained (comparable to the use of powdered surface).

The dry skin lubricity number was 5; the coating adhered satisfactorily to the rubber and no visible flaking was observed.

Examples 2 to 11

Example 1 was repeated, except that the copolymer was replaced by the polymers indicated in the following Table 1:

## Table 1

| Example No. | Polymer | Molar ratio of monomers | Dry skin lubricity number |
|---|---|---|---|
| 2 | HEMA/MAA | 2:1 | 5 |
| 3 | HEMA/MAA | 5:1 | 5 |
| 4 | HEMA/MAA | 10:1 | 5 |
| 5 | HEMA/EHA | 2.5:1 | 5 |
| 6 | HEMA/EHA | 4:1 | 5 |
| 7 | HEMA/EHA | 5:1 | 5 |
| 8 | HEMA/EHA | 10:1 | 5 |
| 9 | HEMA/MAA/EHA | 10:1:0.5 | 5 |
| 10 | HEMA/MAA/EHA | 5:1:1.2 | 5 |
| 11 | HEMA/MAA/BA | 10:1:0.5 | 5 |
| Comparative 1 | HEMA/AA | 2:1 | 4 |
| Comparative 2 | HEMA/AA | 1:1 | 4 |
| Comparative 3 | HEMA/MMA | 2:1 | 4 |
| Comparative 4 | HEMA/MMA | 1:1 | 4 |
| Comparative 5 | HEMA/BA | 5:1 | 4 |
| Comparative 6 | HEMA/BA | 2:1 | 3 |
| Comparative 7 | HEMA/MA | 2:1 | 4 |
| Comparative 8 | HEMA/IA | 2:1 | 4 |
| Comparative 9 | HEMA/EHA | 2:1 | 4 |
| Comparative 10 | HEMA/EHA | 1:1 | 4 |
| Comparative 11 | MMA/VPd | 1:1 | 3 |
| Comparative 12 | HEMA | – | 3-4 |
| Comparative 13 | HEA | – | 3-4 |
| Comparative 14 | VPd | – | 2 |
| Comparative 15 | HPMA | – | 3-4 |
| Comparative 16 | HEMA/HEA | 1:1 | 4 |
| Comparative 17 | HEMA/VPd | 1:1 | 3-4 |
| Comparative 18 | HEMA/HPMA | 1:1 | 4 |
| Comparative 19 | HEA/HPMA | 1:1 | 4 |
| Comparative 20 | HEMA/VPy | 9:1 | 4-5 |

In the above Table, the abbreviations have the following meanings:

| | |
|---|---|
| EHA | 2-ethylhexyl acrylate |
| BA | butyl acrylate |
| AA | acrylic acid |
| MMA | methyl methacrylate |
| MA | methyl acrylate |

IA        itaconic acid
VPd      N-vinyl pyrrolidone
HEA     hydroxyethyl acrylate
HPMA   hydroxypropyl methacrylate
VPy      vinyl pyridine (quaternised)

It will be seen that the dry skin lubricity for each of the Examples according to the invention was better than that obtained in any of the Comparative Examples (the only comparative sample approaching the lubricity of the samples according to the invention was that of Comparative Example 20, when a quaternised copolymer was used).

In each of the Examples according to the invention, the coating adhered satisfactorily with at most very slight flaking. This also applied to the Comparative Examples, except Comparative Example 14 (where the coating was washed off on wet-stripping).

The dry frictional force and the coefficient of friction for the glove of Example 6, the glove of Example 10 and for a conventional powdered glove are given in the following Table 2.

### Table 2

| Glove | Dry frictional force | Coefficient of friction |
|---|---|---|
| Example 6 | 48.7 g | 0.20 |
| Example 10 | 53.1 g | 0.218 |
| Conventional powdered | 78.5 g | 0.323 |

Example 12

Example 1 was repeated except that the copolymer was replaced by a copolymer having the following molar percentage: 80% HEMA, 10% MMA, 10% EHA. A dry skin lubricity number of 5 was obtained.

Examples 13 to 20

Example 1 was repeated, except that the copolymer was replaced by the polymers indicated in the following Table 3.

### Table 3

| Example No. | Molar percentage HEMA | Molar percentage MAA | Molar percentage EHA |
|---|---|---|---|
| 13 | 70 | 20 | 10 |
| 14 | 60 | 10 | 30 |
| 15 | 60 | 20 | 20 |
| 16 | 60 | 30 | 10 |
| 17 | 50 | 30 | 20 |
| 18 | 40 | 30 | 30 |
| 19 | 40 | 40 | 20 |
| 20 | 30 | 60 | 10 |

In each case, a dry skin lubricity number of 5 was obtained.

6

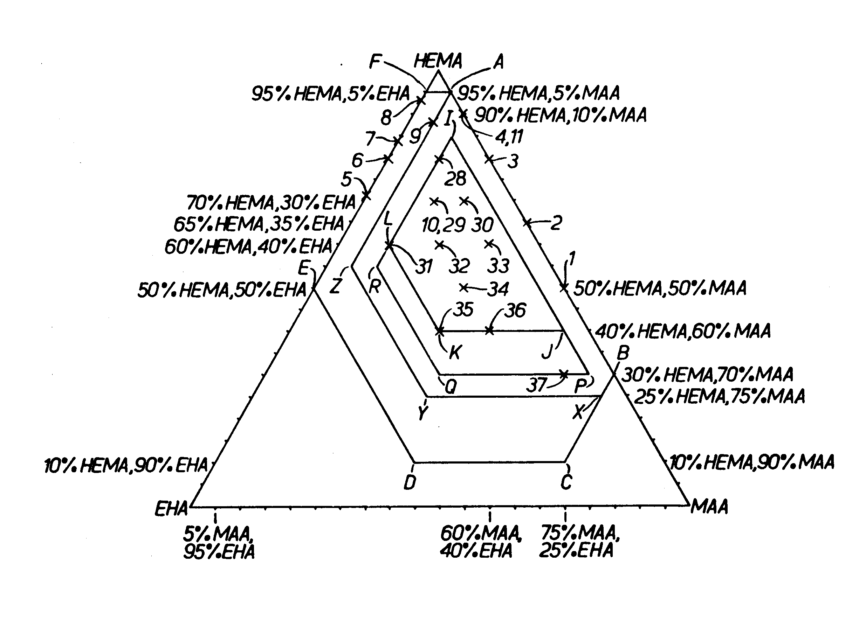

**Claims**

1. A flexible rubber article having a lubricating layer formed from a hydrogel polymer bonded thereto so as to provide a skin-contacting surface of said article, characterised in that the hydrogel polymer is a copolymer of 2-hydroxyethyl methacrylate with methacrylic acid and/or with 2-ethylhexyl acrylate, said copolymer having a composition falling within the area ABCDEF of the attached ternary composition diagram.

2. A flexible rubber article according to claim 1, in which the 2-ethylhexyl acrylate is replaced by another alkyl acrylate or an alkyl methacrylate having an equivalent effect.

3. A flexible rubber article according to claim 1, in which the copolymer has a composition falling within the area ABXYZ of the ternary composition diagram.

4. A flexible rubber article according to claim 1, in which the copolymer has a compositioon falling within the area PQRI of the ternary composition diagram.

5. A process for producing an article according to any of claims 1 to 4, which comprises the steps of:
   (a) forming a rubber article by dipping a suitably shaped former in a rubber latex,
   (b) leaching the rubber article on the former in hot water,
   (c) priming the rubber surface of the article on the former by means of a dilute acid,
   (d) rinsing the primed surface in water or aqueous alkali,
   (e) dipping the rubber article, while still on the former, in a solution of the hydrophilic, hydrogel-forming polymer and a curing agent therefor,
   (f) heating the resulting coating so that the resulting hydrogel polymer is bonded to the rubber and so that the rubber is vulcanised and the polymer is simultaneously cured, and
   (g) stripping the resulting article from the former.

6. A process according to claim 5, which further comprises:
   (h) applying a solution of surfactant material containing silicone to said coating of hydrogel polymer, and
   (i) heating the resulting coating of surfactant material so as to fix the slip properties of the coating.

**Patentansprüche**

1. Flexibler Gummiartikel mit einer aus einem Hydrogelpolymer gebildeten gleitfähigmachenden Schicht, die daran gebunden ist, so daß sie eine die Haut berührende Oberfläche des Artikels bereitstellt, gekennzeichnet dadurch, daß das Hydrogelpolymer ein Copolymer aus 2-Hydroxyethylmethacrylat mit Methacrylsäure und/oder mit 2-Ethylhexylacrylat ist, wobei das Copolymer eine Zusammensetzung hat, die innerhalb des Bereichs ABCDEF des ternären Zusammensetzungsdiagramms im Anhang liegt.

2. Flexibler Gummiartikel nach Anspruch 1, in dem das 2-Ethylhexylacrylat durch ein anderes Alkylacrylat oder ein Alkylmethacrylat ersetzt ist, welches eine gleichwertige Wirkung hat.

3. Flexibler Gummiartikel nach Anspruch 1, in dem das Copolymer eine Zusammensetzung hat, die innerhalb des Bereichs ABXYZ des ternären Zusammensetzungsdiagramms liegt.

4. Flexibler Gummiartikel nach Anspruch 1, in dem das Copolymer eine Zusammensetzung hat, die innerhalb des Bereichs PQRI des ternären Zusammensetzungsdiagramms liegt.

5. Verfahren zum Herstellen eines Artikels nach einem der Ansprüche 1 bis 4, welches die Schritte umfaßt:
   a) Bilden eines Gummiartikels durch Eintauchen eines in geeigneter Weise gestalteten Formkörpers in einen Gummilatex,
   b) Auslaugen des Gummiartikels auf dem Formkörper in heißem Wasser,
   c) Grundieren der Gummioberfläche des Artikels auf dem Formkörper mittels einer verdünnten Säure,
   d) Spülen der grundierten Oberfläche in Wasser oder wäßrigem Alkali,

7

e) Eintauchen des Gummiartikels, der sich immer noch auf dem Formkörper befindet, in eine Lösung des hydrophilen, Hydrogel-bildenden Polymers und eines Härtungsmittels dafür,

f) Erwärmen des daraus hervorgehenden Überzugs, so daß das resultierende Hydrogelpolymer an den Gummi gebunden und so daß der Gummi vulkanisiert und das Polymer gleichzeitig gehärtet wird, und

g) Abstreifen des daraus sich ergebenden Artikels von dem Formkörper.

6. Verfahren nach Anspruch 5, welches weiter umfaßt:

h) Auftragen einer Lösung eines oberflächenaktiven Materials, welches Silicon enthält, auf den Überzug aus Hydrogelpolymer, und

i) Erwärmen des sich ergebenden Überzugs aus oberflächenaktiven Material, so daß die Gleiteigenschaften des Überzugs fixiert werden.

**Revendications**

1. Un article en caoutchouc flexible sur lequel est collée une couche formée d'un polymère d'hydrogel de manière à fournir une surface dudit article en contact avec la peau, caractérisé en ce que le polymère d'hydrogel est un copolymère de méthacrylate de 2-hydroxyéthyle avec l'acide méthacrylique et/ou avec l'acrylate de 2-éthylhexyle, ledit copolymère ayant une composition comprise dans la région ABCDEF du diagramme de composition ternaire ci-annexé.

2. Un article en caoutchouc flexible selon la revendication 1, dans lequel l'acrylate de 2-éthylhexyle est remplacé par un autre acrylate d'alkyle ou un méthacrylate d'alkyle ayant un effet équivalent.

3. Un article de caoutchouc flexible selon la revendication 1, dans lequel le copolymère a une composition comprise dans la région ABXYZ dans le diagramme de composition ternaire.

4. Un article de caoutchouc flexible selon la revendication 1, dans lequel le copolymère a une composition comprise dans la région PQRI dans le diagramme de composition ternaire.

5. Un procédé pour produire un article selon l'une quelconque des revendications 1 à 4, qui comprend les étapes suivantes :

(a) on forme un article de caoutchouc en plongeant un gabarit de forme convenable dans un latex de caoutchouc,

(b) on extrait dans l'eau chaude l'article de caoutchouc sur le gabarit,

(c) on amorce la surface de caoutchouc de l'article sur le gabarit, par exemple au moyen d'un acide dilué,

(d) on rince la surface amorcée dans l'eau ou un alcali aqueux,

(e) on plonge ledit article, toujours sur le gabarit, dans une solution d'un polymère hydrophile formant un hydrogel et d'un agent durcissant pour celui-ci,

(f) on chauffe le revêtement résultant de sorte que le polymère d'hydrogel résultant soit collé au caoutchouc et que le caoutchouc soit vulcanisé et le polymère soit simultanément durci et

(g) on enlève l'article résultant du gabarit.

6. Un procédé selon la revendication 5 qui comprend en outre les étapes suivantes:

(h) on applique une solution d'une substance tensioactive contenant une silicone sur l'article (par exemple par culbutage dans cette solution), facultativement après lavage et

(i) on chauffe le revêtement de substance tensioactive résultant de manière à fixer les propriétés de glissement du revêtement.